Europäisches Patentamt

European Patent Office

Office européen des brevets

(11) Veröffentlichungsnummer : **0 447 355 A1**

# EUROPÄISCHE PATENTANMELDUNG

(21) Anmeldenummer : 91810096.7

(22) Anmeldetag : 12.02.91

(51) Int. Cl.⁵ : **A61F 2/08**

(30) Priorität : 12.03.90 CH 787/90

(43) Veröffentlichungstag der Anmeldung :
18.09.91 Patentblatt 91/38

(84) Benannte Vertragsstaaten :
AT CH DE FR GB IT LI

(71) Anmelder : GEBRÜDER SULZER
AKTIENGESELLSCHAFT
Zürcherstrasse 12
CH-8400 Winterthur (CH)

(72) Erfinder : Frey, Otto, Dr.
Wallrütistrasse 56
CH-8400 Winterthur (CH)
Erfinder : Dittes, Peter
Hasenweidtstrasse 6
CH-8635 Oberdürnten (CH)
Erfinder : Koch, Rudolf
Oberdorfstrasse 229
CH-8267 Berlingen (CH)

(54) Implantat für den menschlichen Körper.

(57) Mindestens der äussere Schlauch (1) eines aus einer Vielzahl von Schläuchen (1) bestehenden Implantates, das als Bänderersatz dient, ist mit einer geschlossene, für Abrieb undurchlässigen Schicht eines Elastomers (3) versehen, dessen Schichtdicke zwischen den Fäden (2) und am Rand 0,1 mm nicht übersteigt.

Auf diese Weise wird ein Austreten von Abrieb in das umgebende Körpergewebe verhindert, ohne dass das mechanische Verhalten, wie Dehnbarkeit, Biegesteifigkeit und/oder Torsionsstabilität, gegenüber einem Implantat ohne Elastomerschicht ungünstig verhändert wird.

## Fig.1

EP 0 447 355 A1

GEBRÜDER SULZER, AKTIENGESELLSCHAFT, WINTERTHUR/SCHWEIZ

## Implantat für den menschlichen Körper

Die Erfindung betrifft ein aus textilen Fäden hergestelltes Implantat für den menschlichen Körper, das aus mehreren Lagen in sich geschlossener, übereinandergezogener Schläuche besteht.

Ein Implantat der vorstehend genannten Art, das vorwiegend als Bandersatz, beispielsweise als Kreuzbandersatz, dient, ist bekannt aus der CH-PS 665 768. Der Kernlose geflochtene Strang dieses bekannten Bandes weist Bereiche unterschiedlicher Biegsamkeit und elastischer Längsdehnbarkeit auf. Bei diesem bekannten Band hat die Praxis besonders für die Teilbereiche mit hoher Biegsamkeit und hoher Längsdehnbarkeit gezeigt, dass nicht ausgeschlossen werden kann, dass die einzelnen, miteinander verflochtenen Fäden der sich konzentrisch "überlagernden" Schläuche abgerieben werden. Dieser Reibungsverschleiss führt zum einen zu im Körper, insbesondere im Kniegelenk, unerwünschten Abriebpartikeln und schwächt zum anderen im Laufe der Zeit die mechanische Festigkeit des Implantates.

Aufgabe der Erfindung ist, den Abrieb an den textilen Fäden des Implantats und/oder den Austritt des Abriefs in den Körper möglichst zu verhindern, ohne dass dabei das mechanische Verhalten des Bandes, d.h. inbesondere seine Dehnungs-, Biege- und Torsionseigenschaften, verändert werden.

Diese Aufgabe wird mit der Erfindung dadurch gelöst, dass mindestens der äusserste Schlauch mit einer für Abrieb undurchlässigen Schicht eines Elastomers versehen ist, dessen Schichtdicke sowohl zwischen den einzelnen Fäden als auch am Rand höchstens 0,1 mm beträgt.

Die bei dem neuen Implantat vorhandene, mindestens den äussersten Schlauch einschliessende, gegen Abrieb undurchlässige Schicht eines Elastomers, das mit Vorteil beispielsweise ein Polyurethan oder ein Silikon sein kann, schliesst das Implantat gegen die Umgebung ab, so dass Abrieb von "inneren" Schläuchen nicht in das Gelenk austreten kann. Die Elastizität des Elastomers und seine geringe Schichtdicke, die beispielsweise licht- oder elektronenmikroskopisch festgestellt wird, bewirken dabei, dass die einzelnen Fäden des oder der durchtränkten Schläuche sich in gleicher Weise wie diejenigen von nicht mit einem Elastomer durchsetzten Schlauch relativ zueinander bewegen können, wobei die Nachgiebigkeit des Elastomers gewährleistet, dass zumindest bei dem äussersten Schlauch die zusammenhängende, für Abrieb undurchlässige Schicht erhalten bleibt.

Selbstverständlich ist es möglich, nicht nur den äussersten Schlauch, sondern auch weiter innen liegende, mit einer zusammenhängenden Elastomerschicht zu versehen, um beispielweise Abrieb praktisch vollständig zu vermeiden; dabei hat es sich bewährt, mindestens einen Teil der Schläuche, die vorteilhafterweise geflochten sind, entweder aus mit einem Elastomer getränkten Fäden herzustellen und nach der Fertigstellung des Geflechtes mindestens einen Teil der bereits mit einem Elastomer "versehenen" Fäden durch thermisches Anschmelzen oder chemisches Anlösen zu einer zusammenhängenden, für Abrieb undurchlässigen Schicht zu verbinden.

Eine weitere vorteilhafte Art, das neue Implantat zu fertigen, besteht darin, dieses zunächst aus elastomerfreien Fäden, die monofil sein oder aus einer Vielzahl von beispielsweise miteinander verzwirnten Filamenten bestehen können - beispielsweise durch Weben, Stricken, Wirken oder Flechten - herzustellen und dann als Ganzes mit einem Elastomer zu durchtränken.

Im folgenden wird die Erfindung anhand von Ausführungsbeispielen im Zusammenhang mit der Zeichnung näher erläutert.

Fig. 1 zeigt schematisch einen Schnitt senkrecht zu Längsachse eines im Querschnitt etwa kreisrunden Bandes;

Fig. 2 gibt, ebenfalls schematisch, einen Schnitt durch eine im wesentlichen einen rechteckigen Querschnitt aufweisende Ausführungsform wieder.

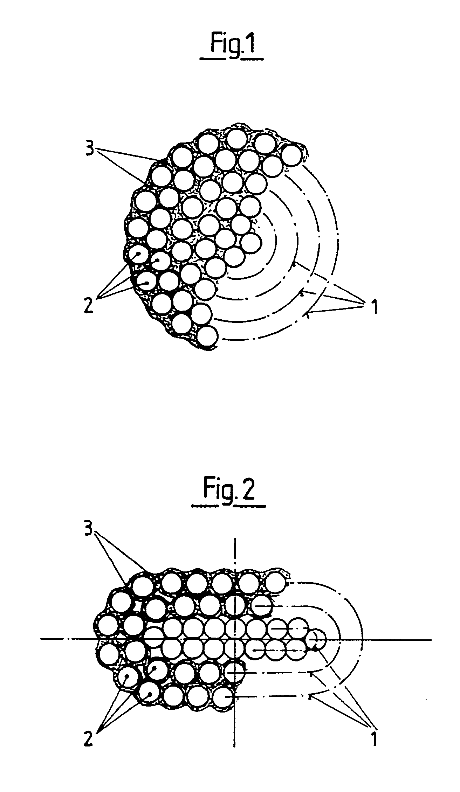

Das Implantat nach Fig. 1 besteht aus einer Anzahl Schläuche 1, die konzentrisch zueinander, wie Jahresringe eines Baumes einander umgeben. Wie erwähnt, können die Schläuche aus monofilen oder multifilen Fäden 2 hergestellt sein und aus Naturfasern, z.B. Seide oder Baumwolle, oder Kunstfasern, wie Polyester, Polyethylen oder Polyethylenterephthalat bestehen. Für einen Kreuzbandersatz im einem Kniegelenk werden dabei etwa 20 bis 30 Schläuche 1 übereinander geschichtet. Im vorliegenden Beispiel bestehen die einzelnen Fäden aus einer Vielzahl, z.B. 40 bis 60 Filamenten, die miteinander verzwirnt sind und beispielsweise Durchmesser von 0,01 bis 0,03 mm haben.

Die einzelnen Schläuche 1 werden im vorliegenden Fall durch Flechten erzeugt. Sie können jedoch auch durch andere Herstellungsverfahren der Textiltechnik, wie Weben, Wirken oder Stricken, gefertigt werden.

Die einzelnen Fäden 2 eines Schlauches 1 und der Schläuche 1 untereinander sind mindstens zum Teil durch "Zwischenschichten" aus einem Elastomer 3 mindestens in den äusseren Bereichen des Implantates zu einer für Abrieb undurchlässige Schicht verbunden, wobei die Schichtdicken des Elastomers 3 maximal 0,1 mm betragen. Damit wird erreicht, dass das mechanische Verhalten des Bandes gegenüber demjenigen eines nicht mit einem Elastomer 3 durchsetzten Bandes nicht ins Gewicht fallend verändert werden.

Als Elastomere 3 werden dabei vorzugsweise Polyurethane oder Silikone verwendet.

Das Implantat nach Fig. 1 ist zunächst aus nicht mit Elastomer 3 durchtränkten Fäden 2 hergestellt und anschliessend als Ganzes getränkt worden. Das Tränken mit dem Elastomer 3 kann dabei beispielsweise wie folgt durchgeführt weren:

Das Geflecht wird zunächst in einer wässrigen Lösung eines zellfreundlichen, im Handel erhältlichen Waschmittels längere Zeit, beispielsweise während 24 Stunden, gewaschen, anschliesend in mehrmals erneuertem Wasser gespült und schliesslich getrocknet.

Um die Benetzbarkeit der Fäden 2 bzw. der Filamente zu erhöhten, wird das Geflecht nun, in einem aus der Färbetechnik von Kunststoff-Fasern bekannten Färbe-Beschleuniger (Carrier) zum Quellen gebracht. Ein derartiger Beschleuniger ist ein organisches Lösungsmittel, beispielsweise Dichlor-Methan oder auch Tetrochlorethylen, Dichlorbenzol oder Trichlorbenzol.

Das gequollene Geflecht wird anschliessend in eine Polyurethan-(PUR)-Lösung getaucht, wobei als Lösungsmittel Dimethylformamid dient und mit dieser Lösung durchtränkt. Nach der Durchtränkung erfolgt ein Trocknen durch strömende Luft in einer Laminar-Flow-Kabine.

Um Lösungsmittelreste zu entfernen, unterwirft man das Implantat abschliessend bei 50° C einem Vakuum von etwa 1 mm HG, wobei es für diese Evakuierung zuvor in eine dampfdurchlässige folie eingepackt worden ist.

Bei dem Ausführungsbeispiel nach Fig. 2 wird das Geflecht mindestens des oder der äusseren Schläuche 1 aus Fäden 2, die mit einem Elastomer 3 bereits durchtränkt worden sind, hergestellt. Solche Fäden 2, die beispielsweise aus Polyester bestehen und mit Polyurethan durchtränkt sind, sind im Handel erhältich. Um mindestens in dem äussersten Schlauch 1 eine zusammenhängende, für Abrieb undurchlässige Elastomerschicht zu erzeugen, wird das Elastomer 3 des fertigen Geflechtes mit einem Lösungsmittel angelöst und anschliessend das Lösungsmittel wieder ausgetrieben.

Es ist jedoch auch möglich, bei Auswahl eines relativ hochschmelzenden Materials für die Fäden 2 und eines niedrig schmelzenden Elastomers 3 das Geflecht bis in den Schmelzbereich des Elastomers 3 zu erhitzen, so dass dieses mindestens an der äusseren Oberfläche schmilzt, von den einzelnen Fäden 2 her ineinander fliesst und beim Abkühlen zu einer geschlossenen Schicht erstarrt.

Dabei können entweder, wie vorstehend, mit dem Elastomer 3 behandelte Fäden 2 aus einem einzigen Material oder multifilamente Fäden 2, die im wesentlichen aus einem hochschmelzenden Material, beispielsweise Polyethylenterephthalat, bestehen und zusätzlich Filamente aus einem niedriger schmelzenden Elastomer-Material, wie beispielsweise Polyethylen enthalten, verwendet werden.

## Patentansprüche

1. Aus textilen Fäden hergestelltes Implantat für den menschlichen Körper, das aus mehreren Lagen in sich geschlossener, übereinandergezogener Schläuche besteht, dadurch gekennzeichnet, dass mindestens der äusserste Schlauch (1) mit einer für Abrieb undurchlässigen Schicht eines Elastomers (3) versehen ist, dessen Schichtdicke sowohl zwischen den einzelnen Fäden als auch am Rand höchstens 0,1 mm beträgt.

2. Implantat nach Anspruch 1, dadurch gekennzeichnet, dass das Implantat Bereiche unterschiedlicher Biegsamkeit und elastischer Dehnbarkeit hat.

3. Implantat nach Anspruch 2, dadurch gekennzeichnet, dass mindestens die Teilbereiche hoher Dehnbarkeit mindestens des äussersten Schlauches (1) mit dem Elastomer (3) versehen sind.

4. Implantat nach einem der Ansprüche 1 bis 3, dadurch gekennzeichnet, dass die Schläuche (1) geflochten sind.

5. Implantat nach einem der Ansprüche 1 bis 4, dadurch gekennzeichnet, dass das Elastomer (3) aus einem Polyurethan besteht.

6. Implantat nach einem der Ansprüche 1 bis 4, dadurch gekennzeichnet, dass das Elastomer (3) aus einem Silikon besteht.

7. Implantat nach einem der Ansprüche 1 bis 6, dadurch gekennzeichnet, dass mindestens der äusserste Schlauch (1) aus mit einem Elastomer (3) getränkten Fäden (2) hergestellt ist.

8. Implantat nach einem der Ansprüche 1 bis 7, dadurch gekennzeichnet, dass es als Ganzes mit einem Elastomer (3) durchtränkt worden ist.

9. Implantat nach einem der Ansprüche 1 bis 4, dadurch gekennzeichnet, dass das Geflecht aus multifilamenten Fäden (2) unterschiedlicher Materialien besteht, von denen mindestens eines einen tiefen Schmelzbereich hat.

10. Implantat nach Anspruch 9, dadurch gekennzeichnet, dass das Geflecht aus Fäden (2) besteht, die Polyethylenterephthalat- und Polyethylen-Filamente enthalten.

## Fig.1

## Fig.2

**Europäisches Patentamt**

# EUROPÄISCHER RECHERCHENBERICHT

Nummer der Anmeldung

EP 91 81 0096

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | FR-A-2 598 315 (J.P.LABOUREAU)<br>*Seite 2, Zeilen 9-22; Seite 3, Zeilen 21-36;<br>Seite 4, Zeilen 10-16,29-32; Seite 5,<br>Zeilen 5-15, Figuren 1,3* | 1,4-7 | A61F2/08 |
| Y |  | 2-3,8-10 |  |
| D,Y | EP-A-0 201 667 (GEBRÜDER SULZER)<br>*Seite 2, Zeile 29 - Seite 3, Zeile 16;<br>Seite 3, Zeilen 23-26; Figuren 1-2* | 2-3 |  |
| A |  | 1 |  |
| Y | DE-A-3 319 953 (L.D.KURTZ)<br>*Seite 9, Zeilen 28-34; Seite 10, Zeile 28 -<br>Seite 11, Zeile 1; Seite 12, Zeilen 2-18;<br>Seite 13, Zeile 25 - Seite 14, Zeile 4;<br>Seite 14, Zeilen 19-29; Seite 18, Zeilen 18-23;<br>Seite 21, Zeilen 23-32* | 8-10 |  |
| A |  | 1,4-5 |  |
| A | EP-A-0 238 263 (RICHARDS MEDICAL)<br>*Seite 5, Zeilen 12-24; Seite 9, Zeilen 21-28*<br>--- | 1,4,6 | **RECHERCHIERTE SACHGEBIETE (Int. Cl.5 )** |
| A | US-A-3 540 452 (F.C.USHER ET AL.)<br>*Spalte 3,Zeilen 34-62; Spalte 3, Zeile 68 -<br>Spalte 4, Zeile 16; Spalte 5, Zeilen 1-28,48-55*<br>----- | 1,5 | A61F<br>A61B<br>A61L |

Der vorliegende Recherchenbericht wurde für alle Patentansprüche erstellt

| Recherchenort | Abschlußdatum der Recherche | Prüfer |
|---|---|---|
| DEN HAAG | 10 MAI 1991 | NICE P. |

**KATEGORIE DER GENANNTEN DOKUMENTE**

X : von besonderer Bedeutung allein betrachtet
V : von besonderer Bedeutung in Verbindung mit einer
      anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
      nach dem Anmeldedatum veröffentlicht worden ist
D : in der Anmeldung angeführtes Dokument
L : aus andern Gründen angeführtes Dokument
........................................................
& : Mitglied der gleichen Patentfamilie, übereinstimmendes
      Dokument

EPO FORM 1503 03.82 (P0463)